# EUROPEAN PATENT APPLICATION

(11) **EP 4 095 149 A1**
(43) Date of publication of application: **30.11.2022**
(21) Application number: 21735129.5
(22) Date of filing: 07.02.2021
(51) Int. Cl.: C07K 14/00, A61K 38/16, A61P 3/10, A61P 3/04, A61P 9/12, A61P 3/06, A61P 1/16, A61P 9/10

(54) **TRIPLE AGONIST FOR GLUCAGON-LIKE PEPTIDE-1 RECEPTOR, GLUCAGON RECEPTOR, AND GASTRIC INHIBITORY POLYPEPTIDE RECEPTOR**

(30) Priority: 23.12.2019 CN 201911341821
(71) Applicant: Vonsun Pharmatech Co., Ltd., SIP Suzhou, Jiangsu 215123 (CN)
(72) Inventor: XIN, Zhongshuai, Suzhou, Jiangsu 215123 (CN); LI, Yifei, Suzhou, Jiangsu 215123 (CN); WEN, Liangzhu, Suzhou, Jiangsu 215123 (CN); ZHONG, Yuanguang, Suzhou, Jiangsu 215123 (CN); CHEN, Huimei, Suzhou, Jiangsu 215123 (CN); ZHANG, Mingyi, Suzhou, Jiangsu 215123 (CN); ZHAO, Mei, Suzhou, Jiangsu 215123 (CN)
(74) Representative: De Clercq & Partners
(86) International application number: PCT/CN2021/075738
(87) International publication number: WO 2021/129894

(57) **Abstract**

Provided is a triple agonist for the glucagon-like peptide-1 receptor (GLP1-R), glucagon receptor (GCGR), and gastric inhibitory polypeptide receptor (GIPR), the triple agonist being based on the amino acid sequence of natural exendin-4 with at least one site having an amino acid substitution, mutation, or chemical modification. The triple agonist of the present invention can be used for the prevention or treatment of metabolic syndrome.

## Description

This application claims the priority of Chinese Patent Application No. 201911341821.0, filed with the China National Intellectual Property Administration on December 23, 2019, and titled with "TRIPLE AGONIST FOR GLUCAGON-LIKE PEPTIDE-1 RECEPTOR, GLUCAGON RECEPTOR, AND GASTRIC INHIBITORY POLYPEPTIDE RECEPTOR".

### FIELD

The present disclosure relates to the biological field, and specifically relates to a triple agonist having activity on GLP1 receptor, GCG receptor and GIP receptor, and related uses thereof.

### BACKGROUND

With the improvement of living standards, obesity and diabetes have become very common metabolic diseases. Obesity is one of the main causes of diabetes^{[1]}. Generally, the progression from obesity to diabetes is as follows: obesity → impaired glucose tolerance → type 2 diabetes → uncontrolled hyperglycemia → diabetic complications. Therefore, for the treatment of diabetes, in addition to lowering blood sugar, controlling body weight is also a factor that must be considered.

Glucagon-like peptide-1 (GLP-1) and its analogs are currently widely used non-insulin hypoglycemic agents. GLP-1 is a polypeptide hormone secreted by intestinal L cells. It acts on islet β cells in a glucose-dependent manner, which promotes the biosynthesis and secretion of insulin, and the increase of insulin has a significant hypoglycemic effect. Furthermore, GLP-1 stimulates the proliferation and differentiation of β cells, inhibits apoptosis of β cells, increases the number of islet β cells, inhibits glucagon secretion, inhibits appetite and food intake, and delays gastric emptying ^{[2]}.

Glucagon (GCG) is a hormone secreted by islet α cells. The functions of GCG and insulin are antagonistic and GCG acts to increase blood sugar. Glucagon has a strong role on promoting glycogenolysis and gluconeogenesis, which can significantly increase blood sugar. In addition, it can activate lipase to promote fat decomposition, and strengthen fatty acid oxidation, which increases the production of ketone bodies ^{[3]}. Therefore, although GCG functions contrarily to the goal of hypoglycemic, it has obvious lipid-lowering and weight-reducing effects.

Gastroinhibitory peptide, or known as glucose-dependent insulin-releasing peptide (GIP), is a type of hormone produced by K cells of the small intestinal mucosa, which can inhibit gastric acid secretion, inhibit pepsinogen secretion, stimulate insulin release, inhibit gastric motility and emptying, stimulate the secretion of small intestinal fluid, and stimulate glucagon secretion^{[4]}.

The receptors of these three hormones belong to the GPCR receptor family, and have similar protein structures and binding mechanisms, which makes it possible to design an agonist targeting two or three receptors. Triple agonists for GLP1-R, GCGR and GIPR can simultaneously exert the functions of three hormones^{[5,6]}. GLP-1 can reduce blood sugar and suppress appetite; GCG can decompose fat and reduce body weight, but it would increase blood sugar; GIP mainly stimulates insulin secretion, but also has the activity of stimulating GCG secretion. The three cooperate with each other to form a feedback mechanism based on the blood sugar concentration, which can not only control blood sugar, but also decompose fat and reduce body weight. For the treatment of diabetes and obesity, triple agonist has significant advantages over GLP-1 analogs along.

### SUMMARY

An object of the present disclosure is to provide a triple agonist of glucagon-like peptide-1 receptor (GLP1-R), glucagon receptor (GCGR) and gastric inhibitory peptide receptor (GIPR).

Another object of the present disclosure is to provide a polynucleotide encoding the triple agonist, a recombinant expression vector comprising the polynucleotide, and a transformant comprising the polynucleotide or the recombinant expression vector.

Yet another object of the present disclosure is to provide a synthesis, separation and purification method for preparing the triple agonist.

Still another object of the present disclosure is to provide a method for chemically modifying the side chain of certain amino acids in the triple agonist.

Yet another object of the present disclosure is to provide a separation and purification method for preparing the triple agonist with chemical modification.

Still another object of the present disclosure is to provide a composition comprising the triple agonist.

Yet another object of the present disclosure is to provide a method for treating a disease of interest, comprising providing the triple agonist or a composition comprising the triple agonist to a subject to be treated.

Still another object of the present disclosure is to provide use of the triple agonist or a composition comprising the triple agonist in the manufacture of a medicament for diabetes mellitus, obesity, hyperlipidemia, hypercholesterolemia, non-alcoholic steatohepatitis, as well as arteriosclerosis, atherosclerosis and coronary heart disease caused by hypercholesterolemia and hyperlipidemia.

In order to achieve the above objects, the technical scheme of the present disclosure is as follows:

The present disclosure provides an isolated polypeptide having agonistic activity on glucagon-like peptide-1 receptor (GLP1-R), glucagon receptor (GCGR) and gastric inhibitory peptide receptor (GIPR), that is, a triple agonist of glucagon-like peptide-1 receptor, glucagon receptor and gastric inhibitory peptide receptor.

In a specific embodiment, the triple agonist of the present disclosure has amino acid substitution, mutation or chemical modification in at least one site on the basis of the natural Exendin-4 amino acid sequence.

In another specific embodiment, the triple agonist of the present disclosure comprises a peptide having an amino acid sequence of general formula 1:

His-Xaa1-Gln-Gly-Thr-Phe-Thr-Ser-Asp-Leu-Ser-Xaa2-Xaa3-Xaa4-Glu-Xaa5-Xaa6-Ala-Xaa7-Arg-Leu-Phe-Ile-Glu-Trp-Leu-Xaa8-Xaa9-Gly-Gly-Pro-Ser-Ser-Gly-Ala-Pro-Pro-Pro-Xaa10

In general formula 1:
Xaa1 is selected from the group consisting of glycine (Gly, G) and aminoisobutyric acid (Aib);
Xaa2 is selected from the group consisting of alanine (Ala, A), arginine (Arg, R) and leucine (Leu, L);
Xaa3 is selected from the group consisting of alanine (Ala, A), glutamic acid (Glu, E) and tyrosine (Tyr, Y);
Xaa4 is selected from the group consisting of methionine (Met, M), leucine (Leu, L) and glutamine (Gln, Q);
Xaa5 is selected from the group consisting of lysine (Lys, K), K-R1 and K-R2, wherein the side chain of K-R1 and K-R2 are chemically modified with lipophilic group;
Xaa6 is selected from the group consisting of glutamic acid (Glu, E) and isoleucine (Ile, I);
Xaa7 is selected from the group consisting of alanine (Ala, A) and valine (Val, V);
Xaa8 is selected from the group consisting of isoleucine (Ile, I) and arginine (Arg, R);
Xaa9 is selected from the group consisting of aspartic acid (Asp, D) and glutamic acid (Glu, E); and
Xaa10 is selected from the group consisting of serine (Ser, S) and amidated serine (S-NH₂).

In another specific embodiment, Xaa5 in the general formula 1 is K-R1 with chemical modification on side chain, wherein K-R1 has a chemical structure of:

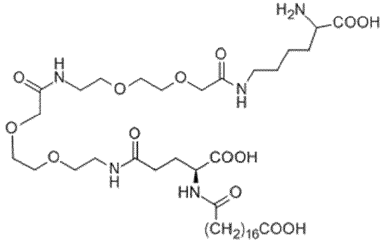

In another specific embodiment, Xaa5 in the general formula 1 is K-R2 with chemical modification on side chain, wherein K-R2 has a chemical structure of:

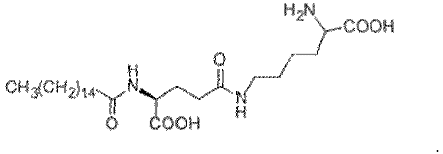

In another specific embodiment, the triple agonist of the present disclosure comprises an amino acid sequence selected from the group consisting of SEQ ID NOs. 1-14 shown in Table 1.

**Table 1**

| **SEQ ID NO.** | **Amino acid sequence** |
|---|---|
| 1 | HXQGT FTSDL SRAME KIAVR LFIEW LREGG PSSGA PPPS |
| 2 | HXQGT FTSDL SRAME KEAVR LFIEW LREGG PSSGA PPPS |
| 3 | HXQGT FTSDL SRAME KEAVR LFIEW LIEGG PSSGA PPPS |
| 4 | HXQGT FTSDL SRAME KEAVR LFIEW LIDGG PSSGA PPPS |
| 5 | HXQGT FTSDL SAAME KIAVR LFIEW LREGG PSSGA PPPS |
| 6 | HXQGT FTSDL SRAME KIAVR LFIEW LIEGG PSSGA PPPS |
| 7 | HXQGT FTSDL SRALE KIAAR LFIEW LIEGG PSSGA PPPS |
| 8 | HXQGT FTSDL SLAQE KEAVR LFIEW LREGG PSSGA PPPS |
| 9 | HXQGT FTSDL SLEQE KIAVR LFIEW LREGG PSSGA PPPS |
| 10 | HXQGT FTSDL SRYLE KEAVR LFIEW LIDGG PSSGA PPPS |
| 11 | HXQGT FTSDL SRAME KIAVR LFIEW LIEGG PSSGA PPPS-NH2 R1 |
| 12 | HXQGT FTSDL SRALE KIAAR LFIEW LIEGG PSSGA PPPS-NH2 R1 |
| 13 | HXQGT FTSDL SRAME KIAVR LFIEW LIEGG PSSGA PPPS-NH2 R2 |
| 14 | HXQGT FTSDL SRALE KIAAR LFIEW LIEGG PSSGA PPPS-NH2 R2 |

Wherein, K-R1 and K-R2 are as defined above.

In another specific embodiment, the triple agonist of the present disclosure has at least one of the following three activities:
I) activating GLP1 receptor;
II) activating glucagon receptor; and
III) activating gastric inhibitory peptide receptor.

In another specific embodiment, the triple agonist with R1 or R2 modification on the K residue has a longer half-life *in vivo* compared to any of native GLP-1, glucagon or gastric inhibitory peptide.

In another specific embodiment, the triple agonist of the present disclosure is amidated at the C-terminus.

Another aspect of the present disclosure is to provide a polynucleotide encoding the triple agonist, a recombinant expression vector comprising the polynucleotide, and a transformant comprising the polynucleotide or the recombinant expression vector.

Yet another aspect of the present disclosure is to provide a method for synthesizing and separating the triple agonist.

Still another aspect of the present disclosure is to provide a method for chemically modifying the side chain of certain amino acids in the triple agonist.

Yet another aspect of the present disclosure is to provide a method for preparing the triple agonist with chemical modification.

Still another aspect of the present disclosure is to provide a composition comprising the triple agonist, and the composition further comprises a pharmaceutically acceptable carrier.

In a specific embodiment, the composition is a pharmaceutical composition.

The present disclosure also provides medicinal use of the triple agonist or the composition comprising the triple agonist.

In another specific embodiment, the present disclosure also provides use of the triple agonist or the composition comprising the triple agonist in the manufacture of a medicament for preventing and/or treating metabolic syndrome.

In another specific embodiment, the metabolic syndrome of the present disclosure includes, but is not limited to, diabetes mellitus, obesity, hypertension, dyslipidemia, hypercholesterolemia, non-alcoholic steatohepatitis, as well as arteriosclerosis, atherosclerosis and coronary heart disease caused by hypercholesterolemia and hyperlipidemia, and the like.

Yet another aspect of the present disclosure is to provide a method for treating a disease of interest, comprising providing the triple agonist or a composition comprising the triple agonist to a subject to be treated.

Beneficial effects of the present disclosure:
The triple agonist of the present disclosure has activities on glucagon-like peptide-1 receptor (GLP1-R), glucagon receptor (GCGR) and gastric inhibitory peptide receptor (GIPR), which have been proven to be therapeutic targets for the above-mentioned metabolic syndrome. Therefore, the triple agonist of the present disclosure can be used for the development of drugs for preventing or treating the above-mentioned metabolic syndrome or clinical treatment of the above-mentioned metabolic syndrome.

### BRIEF DESCRIPTION OF DRAWINGS

FIG. 1 shows the preparative chromatogram of peptide sample of SEQ ID NO: 5;
FIG. 2 shows the UPLC analysis chromatogram of peptide sample of SEQ ID NO: 5;
FIG. 3 shows the mass spectrometry analysis of peptide sample of SEQ ID NO: 5;
FIG. 4 shows the preparative chromatogram of peptide sample of SEQ ID NO: 6;
FIG. 5 shows the UPLC analysis chromatogram of peptide sample of SEQ ID NO: 6;
FIG. 6 shows the mass spectrometry analysis of peptide sample of SEQ ID NO: 6;
FIG. 7 shows the preparative chromatogram of peptide sample of SEQ ID NO: 11;
FIG. 8 shows the UPLC analysis chromatogram of peptide sample of SEQ ID NO: 11;
FIG. 9 shows the mass spectrometry analysis of peptide sample of SEQ ID NO: 11;
FIG. 10 shows the preparative chromatogram of peptide sample of SEQ ID NO: 14;
FIG. 11 shows the UPLC analysis chromatogram of peptide sample of SEQ ID NO: 14;
FIG. 12 shows the mass spectrometry analysis of polypeptide sample of SEQ ID NO: 14.

### DETAILED DESCRIPTION

Hereinafter, the present disclosure will be described in more detail.

Unless otherwise defined herein, scientific and technical terms used in this application shall have the meanings commonly understood by those of ordinary skill in the art. Generally, the terms and methods described herein used in connection with chemistry, molecular biology, cell biology, microbiology, pharmacology, and protein and nucleic acid chemistry are those well-known and commonly used in the art.

All combinations of the various elements disclosed herein are within the scope of the present disclosure. Furthermore, the scope of the present disclosure should not be limited by the specific disclosure provided below.

In addition, the amino acids mentioned herein are abbreviated as follows according to the naming rules of IUPAC-IUB:
Alanine (Ala, A), Arginine (Arg, R), Asparagine (Asn, N), Aspartic acid (Asp, D), Cysteine (Cys, C), Glutamate (Glu, E), Glutamine (Gln, Q), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), Valine (Val, V).

In addition, all amino acid residues in the polypeptide of the present disclosure are preferably in the L configuration.

In addition, the "-NH₂" moiety on the C-terminus of the sequence represents an amide group (-CONH₂) on the C-terminus.

In addition, the unnatural amino acid aminoisobutyric acid (Aib) is used in the sequence of the present disclosure in addition to the natural amino acids.

Compared to the natural glucagon, natural GLP-1 and natural GIP), the triple agonist with activities on glucagon receptor, GLP-1 receptor and GIP receptor may exhibit 0.1% or higher, 1% or higher, 2% or higher, 3% or higher, 4% or higher, 5% or higher, 6% or higher, 7% or higher, 8% or higher, 9% or higher, 10% or higher, 20% or higher, 30% or higher, 40% or higher, 50% or higher, 60% or higher, 70% or higher, 80% or higher, 90% or higher, and 100% or higher *in vitro* activity on one or more of, specifically two or more of, and more specifically all three of the glucagon receptor, GLP-1 receptor and GIP receptor, but not particularly limited thereto.

The method for determining the *in vitro* activity of the triple agonist may refer to Example 4 of the present disclosure, but not particularly limited thereto.

In addition, the triple agonist may be an agonist with increased half-life *in vivo* after R1 or R2 modification on the K residue relative to any of native GLP-1, native glucagon and native GIP, but not particularly limited thereto.

In addition, the polypeptide described herein is an analog of native Exendin-4 selected from variants of native Exendin-4 with at least one amino acid substitution, modification, and a combination thereof in the sequence, but not particularly limited thereto. Wherein the amino acid substitution can include both substitution with an amino acid and substitution with a non-natural compound.

More specifically, the analog of Exendin-4 may be the one in which one or more, two or more, three or more, four or more, five or more, six or more, seven or more, eight or more, nine or more, ten or more, or eleven amino acids at positions 2, 3, 12, 13, 14, 16, 17, 19, 27, 28 and 39 in the natural Exendin-4 amino acid sequence are substituted with other amino acids, but not particularly limited thereto.

The amino acid with which the amino acid is substituted in the above natural Exendin-4 may be selected from alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, valine and aminoisobutyric acid, but not particularly limited thereto.

In addition, the amino acid with which the amino acid is substituted in the above natural Exendin-4 may also be selected from amino acids whose side chains are chemically modified by lipophilic group, specifically, lysine (Lys, K) residue whose side chain is conjugated with a lipophilic substituent. The chemical structures of two lipophilic chemically modified lysine, K-R1 and K-R2, are as follows (the structural formulas of K-R1 and K-R2 in the following examples are the same as here):

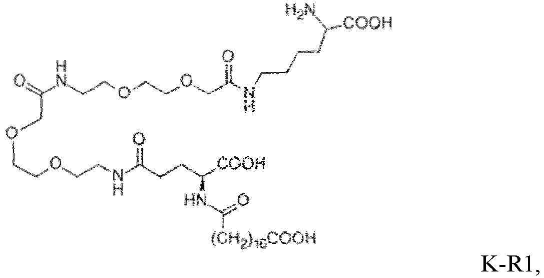

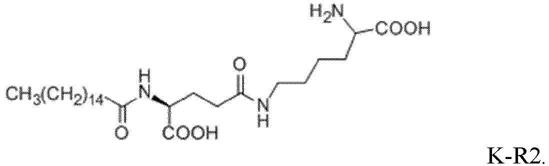

In addition, the polypeptide according to the present disclosure may include all forms of the polypeptide itself, salts thereof (e.g., pharmaceutically acceptable salts thereof), or solvates thereof. Additionally, the polypeptide can be in any pharmaceutically acceptable form. Among them, the kind of salt is not particularly limited, but a salt that is safe and effective for a subject (e.g., mammal) is preferable, but not particularly limited thereto. The term "pharmaceutically acceptable salt" refers to a substance that is effective for its intended use within the limits determined by pharmaco-medical without causing undue toxicity, irritation, allergic reaction, etc. The term "solvate" refers to a complex formed between a polypeptide according to the present disclosure or a salt thereof and a solvent molecule.

In addition, the triple agonist may be an agonist comprising the amino acid sequence selected from the group consisting of SEQ ID NOs. 1-14 in Table 1, or an agonist (substantially) consisting of the amino acid sequence selected from the group consisting of SEQ ID NOs. 1-14 in Table 1, but not limited thereto.

In addition, although described in the present disclosure as a "polypeptide consisting of a specific amino acid sequence", as long as the peptide has the identical or a corresponding activity to a peptide consisting of the corresponding amino acid sequence, it does not exclude those which can be obtained by mutations by addition of nonsense sequences upstream or downstream of the corresponding amino acid sequence, or naturally occurring mutations within the sequence, or silent mutations, and even when sequence additions or mutations exist, they still fall within the scope of the present disclosure.

The triple agonist described herein can significantly activate at least one of glucagon receptor, GLP-1 receptor, and GIP receptor, but not particularly limited thereto. Specifically, the triple agonist can significantly activate GLP-1 receptor, or additionally significantly activate glucagon receptor and/or GIP receptor, but not particularly limited thereto.

The foregoing may apply to other embodiments or aspects of the present disclosure, but not limited thereto.

Another aspect of the present disclosure provides a polynucleotide encoding the triple agonist, a recombinant expression vector comprising the polynucleotide, and a transformant comprising the polynucleotide or recombinant expression vector.

The recombinant vector of the present disclosure is not particularly limited, and any vector known in the art can be used for construction as long as the vector can be replicated in a host cell. The host used in the present disclosure is not particularly limited as long as it can express the polynucleotide of the present disclosure. Examples of suitable hosts include, for example, *Escherichia coli*, *Pichia pastoris*, insect cells, and animal cells, such as CHO cells, and the like.

In addition, the polypeptide of the present disclosure can be synthesized by an automated solid-phase polypeptide synthesizer or produced by a genetic engineering technique.

Specifically, the triple agonist of the present disclosure can be prepared by standard synthetic methods, recombinant expression systems, or any other method known in the art. Thus, the triple agonist of the present disclosure can be synthesized by various methods, including, for example, the methods described below:
I) a method of synthesizing the triple agonist of interest by an automated solid-phase peptide synthesizer, followed by isolating and purifying the final product, or
II) a method of expressing a nucleic acid construct encoding the triple agonist in a host cell and recovering the expression product from the host cell culture, or
   a method of obtaining a target product by ligating the triple agonist fragment obtained by any combination of method I and II, followed by recovering the target triple agonist.

In a more specific example, the triple agonist of interest may be generated by a genetic engineering method, comprising: preparing a fusion gene encoding a fusion protein comprising a fusion partner and the triple agonist, transforming it into a host cell to express the fusion protein, and then isolating the triple agonist by cleaving the fusion protein using a protease or compound.

Yet another aspect of the present disclosure is to provide a method for chemically modifying the side chain of certain amino acids in the triple agonist. Meanwhile, another aspect of the present disclosure is to provide a method for preparing the chemically modified triple agonist.

The chemical modification on the amino acid side chain in the present disclosure specifically refers to the chemical modification on the lysine side chain with lipophilic group. There are mainly two chemical modification of lysine side chain involved in the present disclosure, which are respectively named K-R1 and K-R2, and the specific chemical structure difference is shown in the following schematic diagram. The chemical synthesis method of the lipophilic group side chain R1 and the method for linking to the side chain of lysine by reaction may be referred to CN 201811653280.0. The chemical synthesis method of the lipophilic group side chain R2 in the second case and the method for linking to the side chain of lysine by reaction may be referred to CN1271086C.

Still another aspect of the present disclosure is to provide a composition comprising the isolated triple agonist, in particular, the composition may be a pharmaceutical composition, and more particularly, a pharmaceutical composition for the prevention or treatment of metabolic syndrome.

As used herein, the term "metabolic syndrome" refers to the symptoms of various diseases that occur individually or in combination due to chronic metabolic disorders. In particular, examples of diseases belonging to the metabolic syndrome may include glucose intolerance, diabetes mellitus, obesity, hypertension, dyslipidemia, hypercholesterolemia, non-alcoholic steatohepatitis, as well as arteriosclerosis, atherosclerosis and coronary heart disease caused by hypercholesterolemia and hyperlipidemia, and the like, but not limited thereto.

### Example 1 Synthesis and preparation of triple agonist without chemical modification on side chain

Triple agonists showing activity on GLP-1 receptor, GIP receptor and GCG receptor without chemical modification on side chain were prepared, the amino acid sequences of which are shown in Table 2 below.

**Table 2**

| SEQ ID NO: | Amino acid sequence |
|---|---|
| 1 | HXQGT FTSDL SRAME KIAVR LFIEW LREGG PSSGA PPPS |
| 2 | HXQGT FTSDL SRAME KEAVR LFIEW LREGG PSSGA PPPS |
| 3 | HXQGT FTSDL SRAME KEAVR LFIEW LIEGG PSSGA PPPS |
| 4 | HXQGT FTSDL SRAME KEAVR LFIEW LIDGG PSSGA PPPS |
| 5 | HXQGT FTSDL SAAME KIAVR LFIEW LREGG PSSGA PPPS |
| 6 | HXQGT FTSDL SRAME KIAVR LFIEW LIEGG PSSGA PPPS |
| 7 | HXQGT FTSDL SRALE KIAAR LFIEW LIEGG PSSGA PPPS |
| 8 | HXQGT FTSDL SLAQE KEAVR LFIEW LREGG PSSGA PPPS |
| 9 | HXQGT FTSDL SLEQE KIAVR LFIEW LREGG PSSGA PPPS |
| 10 | HXQGT FTSDL SRYLE KEAVR LFIEW LIDGG PSSGA PPPS |

| | |
|---|---|
| Note: In the sequences set forth in Table 2, X represents the unnatural amino acid aminoisobutyric acid (Aib). | |

### Synthesis of polypeptides without chemical modification on side chain

Fmoc-AA-Wang resin with different amino acids was used as the starting material, and deprotection coupling was carried out according to the sequence. The molar ratio of coupling materials was: Fmoc-AA-Wang resin/Fmoc-AA-OH/HOBT/DIC=1/5/6/6; the activation solvent was DMF; the coupling time was 1.5h; the deprotection was performed using 20% pip/DMF for 5+15 min at room temperature. The synthesis was carried out according to the VS-P and VS-C series polypeptide sequences until the completion of reaction, and the peptide resin was obtained after drying.

A lysis buffer with a ratio of TFA/PhSMe/PhOMe/EDT=90/5/2/3 was prepared and added to the peptide resin, 1 g of peptide resin per 10 ml of lysis buffer. The peptide resin was added at low temperature to react for 10 min first, and then transferred to room temperature for 2 h. After filtration, ice cold methyl tert-butyl ether was added to the filtrate for precipitation, 1 ml of lysate per 10 ml of ice cold methyl tert-butyl ether, then washed 5 times using the same amount of ice cold methyl tert-butyl ether, and centrifuged to obtain a crude polypeptide.

### Treatment of synthesized samples

The synthesized crude peptides were completely vacuum-dried, dissolved in an aqueous solution of triethylamine phosphate buffer (pH=1.8-3.0), prepared into a sample solution of about 5 mg/ml, and filtered with a 0.45-micron membrane. The solution was adjusted for pH to the isoelectric point of the sample with dilute ammonia solution, and then centrifuged to collect the precipitates. The precipitates were redissolved in triethylamine phosphate buffer and filtered with a 0.45-micron filter for later use.

### Purification of peptides

Gradient elution was carried out using 90% 20 mM triethylamine phosphate buffer (pH=1.80) + 10% acetonitrile as phase A, 90% acetonitrile + 10% 20 mM triethylamine phosphate buffer as phase B, 20^{∗} 250mm C18 reversed-phase column, at a flow rate of 10 ml/min. Target peaks were collected. Each fraction was tested and analyzed. The qualified fractions were combined, and for unqualified fractions, the purification operation was repeated, or other elution system was used for purification, and then the fractions meeting the purity requirements were collected and combined. The collected fractions were evaporated to remove acetonitrile, then settled with dilute ammonia water and centrifuged to collect precipitates. The precipitates were washed with water, dissolved in dilute aqueous acetic acid solution and filtered. The filtrate was lyophilized, weighed and calculated the yield, ≥40%. The peptides were frozen in aliquots for storage. The corresponding mass spectrometry data were analyzed according to the molecular weight of the target polypeptide, and the theoretical value and the measured value ranged within plus or minus 1.

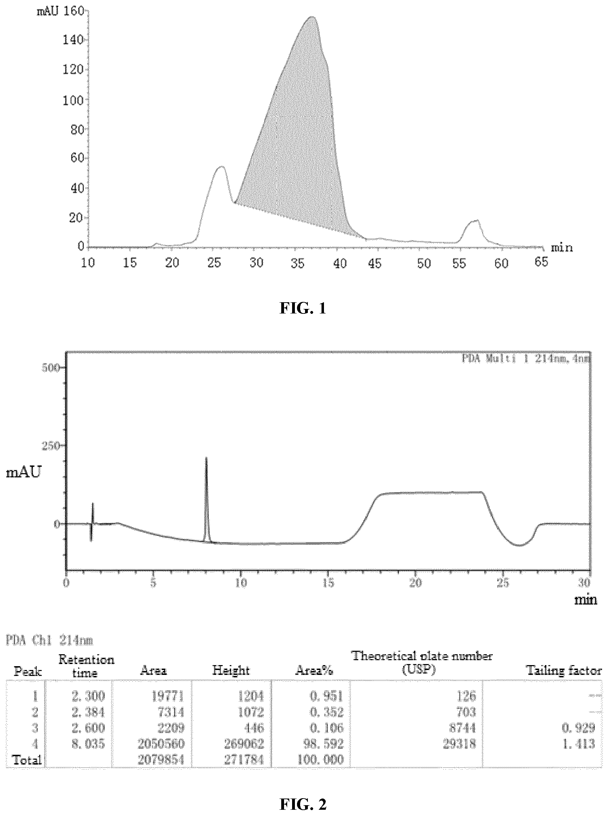

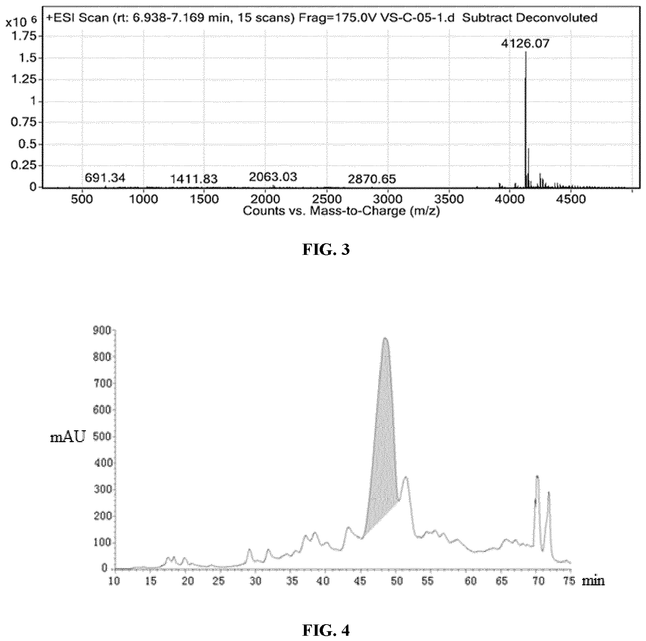

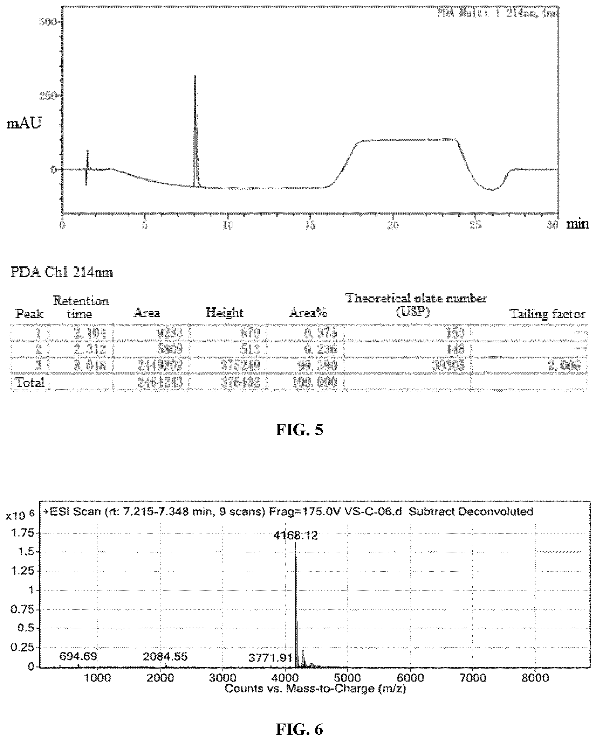

FIGs. 1-6 show the preparative reverse chromatograms of the peptides, the UPLC analysis chromatograms and mass spectrometry analysis of the pure products after lyophilization.

### Example 2 Synthesis and preparation of triple agonist chemically modified with R1 side chain

The amino acid sequences of the triple agonists chemically modified with R1 side chain are shown in the table below:

**Table 3**

| **SEQ ID NO.** | **Amino acid sequence** |
|---|---|
| 11 | HXQGT FTSDL SRAME KIAVR LFIEW LIEGG PSSGA PPPS-NH2 R1 |
| 12 | 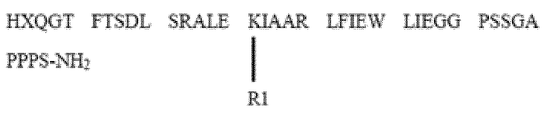 HXQGT FTSDL SRALE KIAAR LFIEW LIEGG PSSGA PPPS-NH2 R1 |

| | |
|---|---|
| Note: In the sequences set forth in Table 3, X represents the unnatural amino acid aminoisobutyric acid (Aib); -NH₂ represents the C-terminal of the amidated polypeptide (-CONH₂); K-R1 and K-R2 represent two chemical modifications on the side chain of lysine with lipophilic group, respectively, and the specific chemical structures are described above. | |

### Synthesis of polypeptides with R1 side chain

Rink Amide-MBHA resin was used as the starting material, and deprotection coupling was carried out according to the synthetic sequence. The molar ratio of coupling materials was: Rink Amide-MBHA resin/Fmoc-AA-OH/HOBT/DIC=1/5/6/6, and the activation solvent was DMF; the coupling time was 1.5h; the deprotection was performed using 20% pip/DMF for 5+15 min at room temperature. The synthesis was carried out according to the VS-CA series polypeptide sequence until the completion of reaction, and the peptide resin was obtained after drying.

A lysis buffer with a ratio of TFA/PhSMe/PhOMe/EDT=90/5/2/3 was prepared and added to the peptide resin, 1 g of peptide resin per 10 ml of lysis buffer. The peptide resin was added at low temperature to react for 10 min first, and then transferred to room temperature for 2 h. After filtration, ice cold methyl tert-butyl ether was added to the filtrate for precipitation, 1 ml of lysate per 10 ml of ice cold methyl tert-butyl ether once, washed 5 times using the same amount of ice cold methyl tert-butyl ether, and centrifuged to obtain crude polypeptide.

The crude polypeptide was added to 0.25 M triethylamine phosphate buffer solution (pH = 11.65) and stirred to dissolve (concentration of 10 mg polypeptide/ml buffer); cooled to 0-5°C in an ice bath, and added with 3.0 eq of R1 side chain (100 mg/ml) which was dissolved in N-methylpyrrolidone. The reaction was performed at 0-5°C for 10 min to obtain a crude peptide. For details on the synthesis method of R1 side chain, reference could be made to Patent Application CN 201811653280.0.

### Treatment of synthesized samples

The synthesized crude peptides were completely vacuum-dried, dissolved in a dilute ammonium bicarbonate solution, prepared into a sample solution of about 5 mg/ml, and filtered with a 0.45-micron membrane for later use.

### Purification of peptides

Gradient elution was carried out using an aqueous solution containing 1% acetic acid and 20% ethanol was used as phase A, an aqueous solution containing 1% acetic acid and 80% ethanol as phase B, 20^{∗} 250mm PS reversed-phase column and at a flow rate of 10 ml/min. Target peaks were collected. Each fraction was tested and analyzed. The qualified fractions were combined, and for unqualified fractions, the purification operation was repeated with C18 reversed-phase column, or other elution system was used for purification, and then the fractions meeting the purity requirements were collected and combined. The collected fractions were evaporated to remove organic solvent, then settled with a dilute aqueous acetic acid solution and centrifuged to collect precipitates. The precipitates were washed with water, redissolved, filtered, lyophilized, weighed and calculated the yield, ≥70%. The peptides were frozen in aliquots for storage. The corresponding mass spectrometry data were analyzed according to the molecular weight of the target polypeptide, and the theoretical value and the measured value ranged within plus or minus 1.

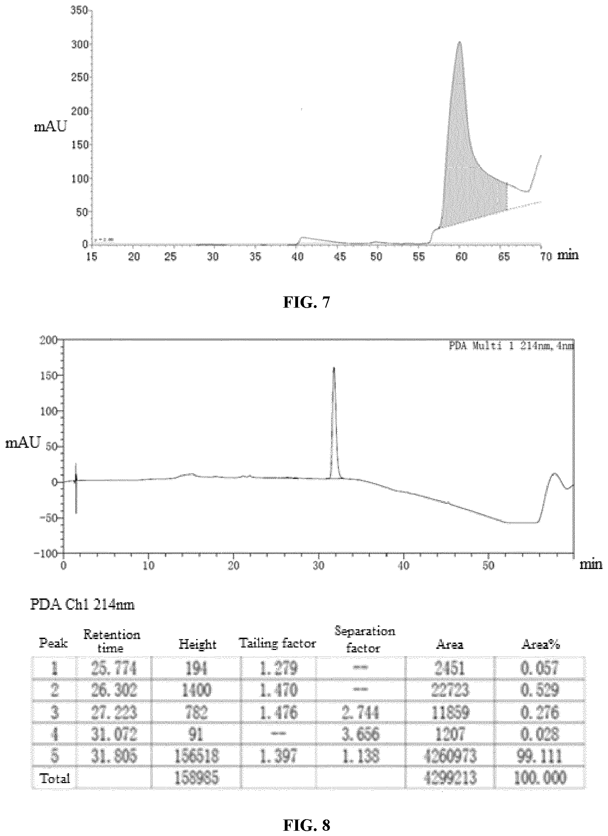

FIGs. 7-9 show the preparative reverse chromatograms of the peptide, the UPLC analysis chromatograms and mass spectrometry analysis of the pure product after lyophilization.

### Example 3 Synthesis and preparation of triple agonist chemically modified with R2 side chain

**Table 4**

| **SEQ ID NO.** | **Amino acid sequence** |
|---|---|
| 13 | HXQGT FTSDL SRAME KIAVR LFIEW LIEGG PSSGA PPPS-NH2 R2 |
| 14 | HXQGT FTSDL SRALE KIAAR LFIEW LIEGG PSSGA PPPS-NH2 R2 |

| | |
|---|---|
| Note: In the sequences set forth in Table 4, X represents the unnatural amino acid aminoisobutyric acid (Aib); -NH₂ represents the C-terminal of the amidated polypeptide (-CONH₂); K-R1 and K-R2 represent two chemical modifications on the side chain of lysine with lipophilic group, respectively, and the specific chemical structures are described above. | |

### Synthesis of polypeptides with R2 side chain

Rink Amide-MBHA resin was used as the starting material, and deprotection coupling was carried out according to the synthetic sequence. The molar ratio of coupling materials was: Rink Amide-MBHA resin/Fmoc-AA-OH/HOBT/DIC=1/5/6/6; the activation solvent was DMF; the coupling time was 1.5h; the deprotection was performed using 20% pip/DMF for 5+15 min at room temperature. The synthesis was carried out according to the VS-CA series polypeptide sequence until the end, and the peptide resin was obtained after drying.

A lysis buffer with a ratio of TFA/PhSMe/PhOMe/EDT=90/5/2/3 was prepared and added to the peptide resin, 1 g of peptide resin per 10 ml of lysis buffer. The peptide resin was added at low temperature to react for 10 min first, and then transferred to room temperature for 2 h. After filtration, ice cold methyl tert-butyl ether was added to the filtrate for precipitation, 1 ml of lysate per 10 ml of ice cold methyl tert-butyl ether, washed 5 times using the same amount of ice cold methyl tert-butyl ether, and centrifuged to obtain crude polypeptide.

The crude polypeptide was added to 0.25 M triethylamine phosphate buffer solution (pH = 11.65) and stirred to dissolve (concentration of 10 mg polypeptide/ml buffer); cooled to 0-5°C in an ice bath, and added with 1.5 eq of R2 side chain (100 mg/ml) which was dissolved in N-methylpyrrolidone. The reaction was performed at 0-5°C for 10 min to obtain a crude peptide. For details on the synthesis method of R2 side chain, reference could be made to Patent CN1271086C.

### Treatment of synthesized samples

The synthesized crude peptides were completely vacuum-dried, prepared into a sample solution of about 5 mg/ml with purified water, adjusted for pH to 7.0-9.0 with dilute ammonia water, and filtered with a 0.45-micron membrane for later use.

### Purification of polypeptides

Gradient elution was carried out using an aqueous solution containing 80 mM ammonium acetate as phase A, acetonitrile as phase B, 20^{∗} 250mm C18 reversed-phase column and at a flow rate of 10 ml/min. Target peaks were collected. Each fraction was tested and analyzed. The qualified fractions were combined, and for unqualified fractions, the purification operation was repeated with other types of columns, or other elution system was used for purification, and then the fractions meeting the purity requirements were collected and combined. The collected fractions were evaporated to remove organic solvent, then settled with a dilute aqueous acetic acid solution and centrifuged to collect precipitates. The precipitates were washed with water, redissolved, filtered, lyophilized, weighed and calculated the yield, ≥70%. The purified peptides were labeled and frozen in aliquots for storage. The corresponding mass spectrometry data were analyzed according to the molecular weight of the target polypeptide, and the theoretical value and the measured value ranged within plus or minus 1.

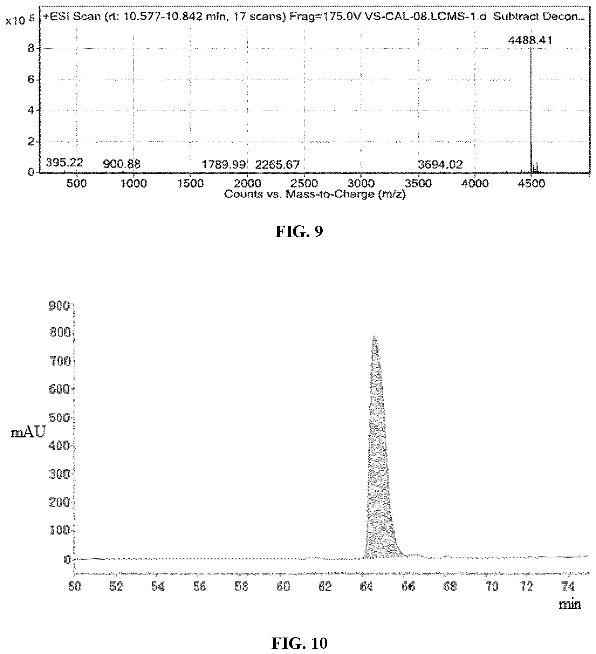

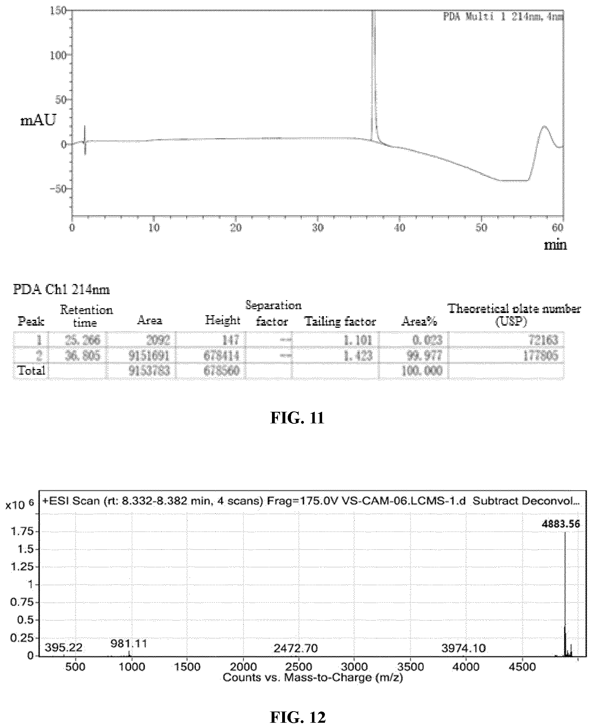

FIGs. 10-12 show the preparative reverse chromatograms of the polypeptide, the UPLC analysis chromatograms and mass spectrometry analysis of the pure product after lyophilization.

### Example 4 In vitro activity assay of the triple agonists

The activities of the triple agonists prepared in Examples 1, 2, and 3 were determined by a method of determining cellular activity *in vitro* using cell lines in which GLP-1 receptor, GCG receptor, and GIP receptor were transformed, respectively. The GLP-1 receptor was expressed in human kidney epithelial H293 cells, while the GCG receptor and GIP receptor were expressed in Chinese hamster ovary cell CHO K1. The activity was determined using transformed cell lines respectively. For the determination of the *in vitro* activity of the triple agonists, the cAMP-GS Dynamic Kit from Cisbio was used, and the test method was HTRF (Homogeneous Time-Resolved Fluorescence), which required a microplate reader equipped with an HTRF module.

To measure the GLP-1 activity of the triple agonists prepared in Examples 1, 2, and 3, the triple agonists prepared in Example 1 was serially diluted 3-fold from 3,000 nM to 1 nM. As a positive control, native GLP-1 was also serially diluted 3-fold from 3,000 nM to 1 nM. In order to test the GLP-1R activity of the triple agonists, the cultured GLP-1R-expressing human renal epithelial cells H293 were digested, centrifuged, collected, diluted to a concentration of 600,000 cells/ml and added to detection wells of a 384-well plate at 5 µl/well. Then cells were added with the diluted triple agonist at 5 µl/well, mixed, and incubated at 37°C for 90 min. According to the protocol of the CAMP-GS Dynamic Kit, the diluted cAMP-d2 reagent and cAMP-Cryptate reagent were added to the detection wells of the 384-well plate sequentially, mixed well, and placed at room temperature for 60 min in the dark. Finally, the 384-well plate was placed in a microplate reader equipped with an HTRF module, EC₅₀ values were calculated from the accumulated cAMP, and these values were compared with each other. Relative titers compared to human GLP-1 are shown in Table 2 below.

To determine the GCG activity of the triple agonists prepared in Examples 1, 2, and 3, the triple agonists prepared in Example 1 was serially diluted 3-fold from 3,000 nM to 1 nM. As a positive control, native GCG was serially diluted 3-fold from 1,000 nM to 0.5 nM. In order to test the GCG activity of the triple agonists, the cultured GCGR-expressing Chinese hamster ovary cells CHO K1 were digested, centrifuged, collected, diluted to a concentration of 600,000 cells/ml and added to detection wells of a 384-well plate at 5 µl/well. Then cells were added with the diluted triple agonist at 5 µl/well, mixed, and incubated at 37°C for 90 min. According to the protocol of the CAMP-GS Dynamic Kit, the diluted cAMP-d2 reagent and cAMP-Cryptate reagent were added to the detection wells of the 384-well plate sequentially, mixed well, and placed at room temperature for 60 min in the dark. Finally, the 384-well plate was placed in a microplate reader equipped with an HTRF module, EC₅₀ values were calculated from the accumulated cAMP, and these values were compared with each other. Relative titers compared to human GCG are shown in Table 2 below.

To determine the GIP activity of the triple agonists prepared in Examples 1, 2, and 3, the triple agonists prepared in Example 1 was serially diluted 3-fold from 1,000 nM to 0.5 nM. As a positive control, native GIP was serially diluted 3-fold from 30 nM to 0.25 nM. In order to test the GIPR activity of the triple agonists, the cultured GIPR-expressing Chinese hamster ovary cells CHO K1 were digested, centrifuged, collected, diluted to a concentration of 600,000 cells/ml and added to detection wells of a 384-well plate at 5 µl/well. Then cells were added with the diluted triple agonist at 5 µl/well, mixed, and incubated at 37°C for 90 min. According to the protocol of the CAMP-GS Dynamic Kit, the diluted cAMP-d2 reagent and cAMP-Cryptate reagent were added to the detection wells of the 384-well plate sequentially, mixed well, and placed at room temperature for 60 min in the dark. Finally, the 384-well plate was placed in a microplate reader equipped with an HTRF module, EC₅₀ values were calculated from the accumulated cAMP, and these values were compared with each other. Relative titers compared to human GIP are shown in Table 5 below.

The results of the assay show that although not always more active than single GLP-1, GCG or GIP receptor agonists, the polypeptides and polypeptide-modified compounds obtained in Examples 1, 2 and 3 exhibit activity for all three GLP-1 receptors, GCG receptors and GIP receptors, which was in line with the characteristics of the triple agonist described herein. Since these three receptors have been proven to be therapeutic targets for metabolic syndromes such as diabetes mellitus, obesity, hypertension, dyslipidemia, hypercholesterolemia, non-alcoholic steatohepatitis, as well as arteriosclerosis, atherosclerosis and coronary heart disease caused by hypercholesterolemia and hyperlipidemia, while the existing drugs on the market are single agonists for one of these three targets, so the triple agonist of the present disclosure can be used for the development of therapeutic drugs for these metabolic syndrome, and for clinical treatment. Data from *in vitro* studies initially support this effect, which will provide the basis for the development of a new generation of therapeutics for these diseases.

**Table 5**

| | *In vitro* activity relative to native polypeptide (%) | | |
|---|---|---|---|
| SEQ ID NO: | Relative to GLP-1 | Relative to GCG | Relative to GIP |
| 1 | 64.69 | 1.33 | 3.99 |
| 2 | 103.50 | 25.08 | 34.02 |
| 3 | 67.65 | 12.23 | 12.11 |
| 4 | 71.38 | 2.17 | 7.37 |
| 5 | 38.40 | 0.72 | 1.99 |
| 6 | 115.64 | 0.89 | 3.27 |
| 7 | 47.48 | 18.26 | 5.74 |
| 8 | 123.21 | 1.21 | 2.02 |
| 9 | 136.18 | 20.10 | 95.77 |
| 10 | 71.63 | 21.84 | 94.68 |
| 11 | 190.51 | 3.89 | 2.53 |
| 12 | 241.57 | 5.98 | 2.43 |
| 13 | 217.16 | 16.15 | 9.83 |
| 14 | 57.00 | 0.49 | 3.38 |

### References:

1. Toplak, H.; Hoppichler, F.; Wascher, T. C.; Schindler, K.; Ludvik, B., [Obesity and type 2 diabetes]. Wien Klin Wochenschr 2016, 128 Suppl 2, S196-200.
2. Muller, T. D.; Finan, B.; Bloom, S. R.; D'Alessio, D.; Drucker, D. J.; Flatt, P. R.; Fritsche, A.; Gribble, F.; Grill, H. J.; Habener, J. F.; Holst, J. J.; Langhans, W.; Meier, J. J.; Nauck, M. A.; Perez-Tilve, D.; Pocai, A.; Reimann, F.; Sandoval, D. A.; Schwartz, T. W.; Seeley, R. J.; Stemmer, K.; Tang-Christensen, M.; Woods, S. C.; DiMarchi, R. D.; Tschop, M. H., Glucagon-like peptide 1 (GLP-1). Mol Metab 2019, 30, 72-130.
3. Heppner, K. M.; Habegger, K. M.; Day, J.; Pfluger, P. T.; Perez-Tilve, D.; Ward, B.; Gelfanov, V.; Woods, S. C.; DiMarchi, R.; Tschop, M., Glucagon regulation of energy metabolism. Physiol Behav 2010, 100 (5), 545-8.
4. Baggio, L. L.; Drucker, D. J., Biology of incretins: GLP-1 and GIP. Gastroenterology 2007, 132 (6), 2131-57.
5. Finan, B.; Yang, B.; Ottaway, N.; Smiley, D. L.; Ma, T.; Clemmensen, C.; Chabenne, J.; Zhang, L.; Habegger, K. M.; Fischer, K.; Campbell, J. E.; Sandoval, D.; Seeley, R. J.; Bleicher, K.; Uhles, S.; Riboulet, W.; Funk, J.; Hertel, C.; Belli, S.; Sebokova, E.; Conde-Knape, K.; Konkar, A.; Drucker, D. J.; Gelfanov, V.; Pfluger, P. T.; Muller, T. D.; Perez-Tilve, D.; DiMarchi, R. D.; Tschop, M. H., A rationally designed monomeric peptide triagonist corrects obesity and diabetes in rodents. Nat Med 2015, 21 (1), 27-36.
6. Jall, S.; Sachs, S.; Clemmensen, C.; Finan, B.; Neff, F.; DiMarchi, R. D.; Tschop, M. H.; Muller, T. D.; Hofmann, S. M., Monomeric GLP-1/GIP/glucagon triagonism corrects obesity, hepatosteatosis, and dyslipidemia in female mice. Mol Metab 2017, 6 (5), 440-446.

## Claims

1. A triple agonist of glucagon-like peptide-1 receptor (GLP1-R), glucagon receptor (GCGR) and gastric inhibitory peptide receptor (GIPR), comprising a peptide having an amino acid sequence of general formula 1:
His-Xaa1-Gln-Gly-Thr-Phe-Thr-Ser-Asp-Leu-Ser-Xaa2-Xaa3-Xaa4-Glu-Xaa5-Xaa6-Ala-Xaa7-Arg-Leu-Phe-Ile-Glu-Trp-Leu-Xaa8-Xaa9-Gly-Gly-Pro-Ser-Ser-Gly-Ala-Pro-Pro-Pro-Xaa10
wherein, in general formula 1:
Xaa1 is selected from the group consisting of glycine (Gly, G) and aminoisobutyric acid (Aib);
Xaa2 is selected from the group consisting of alanine (Ala, A), arginine (Arg, R) and leucine (Leu, L);
Xaa3 is selected from the group consisting of alanine (Ala, A), glutamic acid (Glu, E) and tyrosine (Tyr, Y);
Xaa4 is selected from the group consisting of methionine (Met, M), leucine (Leu, L) and glutamine (Gln, Q);
Xaa5 is selected from the group consisting of lysine (Lys, K), K-R1 and K-R2, wherein the side chain of K-R1 and K-R2 are chemically modified with lipophilic group;
Xaa6 is selected from the group consisting of glutamic acid (Glu, E) and isoleucine (Ile, I);
Xaa7 is selected from the group consisting of alanine (Ala, A) and valine (Val, V);
Xaa8 is selected from the group consisting of isoleucine (Ile, I) and arginine (Arg, R);
Xaa9 is selected from the group consisting of aspartic acid (Asp, D) and glutamic acid (Glu, E); and
Xaa10 is selected from the group consisting of serine (Ser, S) and amidated serine (S-NH₂).

2. The triple agonist according to claim 1, wherein K-R1 has a chemical structure of: 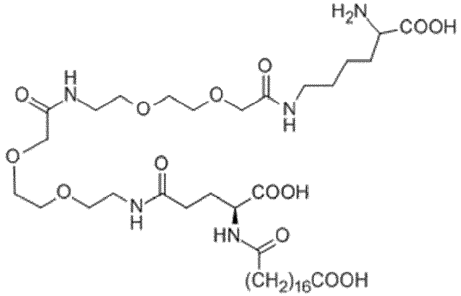 and K-R2 has a chemical structure of:

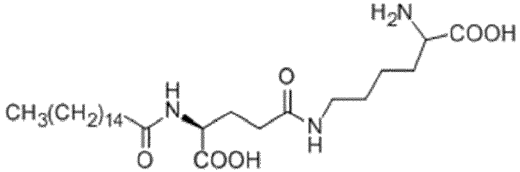

3. The triple agonist according to claim 1, wherein the amino acid sequence is selected from the group consisting of SEQ ID NOs. 1-14 in the following table:
| **SEQ ID NO.** | **Amino acid sequence** |
|---|---|
| 1 | HXQGT FTSDL SRAME KIAVR LFIEW LREGG PS SGA PPPS |
| 2 | HXQGT FTSDL SRAME KEAVR LFIEW LREGG PSSGA PPPS |
| 3 | HXQGT FTSDL SRAME KEAVR LFIEW LIEGG PSSGA PPPS |
| 4 | HXQGT FTSDL SRAME KEAVR LFIEW LIDGG PSSGA PPPS |
| 5 | HXQGT FTSDL SAAME KIAVR LFIEW LREGG PSSGA PPPS |
| 6 | HXQGT FTSDL SRAME KIAVR LFIEW LIEGG PSSGA PPPS |
| 7 | HXQGT FTSDL SRALE KIAAR LFIEW LIEGG PSSGA PPPS |
| 8 | HXQGT FTSDL SLAQE KEAVR LFIEW LREGG PSSGA PPPS |
| 9 | HXQGT FTSDL SLEQE KIAVR LFIEW LREGG PSSGA PPPS |
| 10 | HXQGT FTSDL SRYLE KEAVR LFIEW LIDGG PSSGA PPPS |
| 11 | HXQGT FTSDL SRAME KIAVR LFIEW LIEGG PSSGA PPPS-NH2 R1 |
| 12 | HXQGT FTSDL SRALE KIAAR LFIEW LIEGG PSSGA PPPS-NH2 R1 |
| 13 | 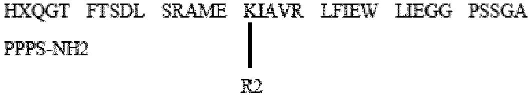 HXQGT FTSDL SRAME KIAVR LFIEW LIEGG PSSGA PPPS-NH2 R2 |
| 14 | HXQGT FTSDL SRALE KIAAR LFIEW LIEGG PSSGA PPPS-NH2 R2 |
wherein, K-R1and K-R2 are as defined in claim 2.

4. A polynucleotide encoding the triple agonist according to any one of claims 1-3.

5. A recombinant expression vector or transformant comprising the triple agonist according to any one of claims 1-3.

6. A composition comprising the triple agonist according to any one of claims 1-3, further comprising a pharmaceutically acceptable carrier.

7. Use of the triple agonist according to any one of claims 1-3, the polynucleotide according to claim 4, the recombinant expression vector or transformant according to claim 5, the composition according to claim 6 in the treatment and/or prevention of metabolic syndrome.

8. The use according to claim 7, wherein the metabolic syndrome comprises diabetes mellitus, obesity, hypertension, dyslipidemia, hypercholesterolemia, non-alcoholic steatohepatitis, and arteriosclerosis and coronary heart disease caused by hypercholesterolemia and hyperlipidemia.
